(19) Europäisches Patentamt
European Patent Office
Office européen des brevets

(11) Veröffentlichungsnummer: **0 076 382**
**A2**

(12) # EUROPÄISCHE PATENTANMELDUNG

(21) Anmeldenummer: 82107947.2

(22) Anmeldetag: 30.08.82

(51) Int. Cl.³: **C 07 C 103/365**
C 07 C 103/37, C 07 D 307/68
C 07 D 261/18, C 07 D 275/02
C 07 D 277/56, C 07 D 285/06
C 07 D 285/12, A 01 N 37/22
A 01 N 43/80

(30) Priorität: 05.09.81 DE 3135238

(43) Veröffentlichungstag der Anmeldung:
13.04.83 Patentblatt 83/15

(84) Benannte Vertragsstaaten:
AT BE CH DE FR GB IT LI NL SE

(71) Anmelder: BASF Aktiengesellschaft
Carl-Bosch-Strasse 38
D-6700 Ludwigshafen(DE)

(72) Erfinder: Zeeh, Bernd, Dr.
Thorwaldsenstrasse 5
D-6700 Ludwigshafen(DE)

(72) Erfinder: Rentzea, Costin, Dr.
Neuenheimer Landstrasse 72
D-6900 Heidelberg(DE)

(72) Erfinder: Ammermann, Eberhard, Dr.
Sachsenstrasse 3
D-6700 Ludwigshafen(DE)

(72) Erfinder: Pommer, Ernst-Heinrich, Dr.
Berliner Platz 7
D-6703 Limburgerhof(DE)

(54) **N-Alkinyl-N-naphthylamide, Verfahren zu ihrer Herstellung und diese enthaltende Fungizide.**

(57) Die vorliegende Erfindung betrifft N-Alkinyl-naphthylamide der Formel

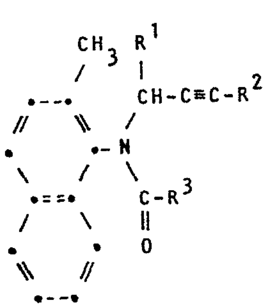

worin $R^1$, $R^2$ und $R^3$ die in der Beschreibung angegebene Bedeutung besitzen, deren Herstellung und Verwendung bei der Fungi-Bekämpfung.

EP 0 076 382 A2

Croydon Printing Company Ltd.

N-Alkinyl-naphthylamide, Verfahren zu ihrer Herstellung und diese enthaltende Fungizide

Die vorliegende Erfindung betrifft neue N-Alkinyl-naphthylamide, Verfahren zu ihrer Herstellung und Fungizide, die diese Verbindungen als Wirkstoffe enthalten.

Es ist bereits bekannt, daß Zink-ethylen-1,2-bisdithiocarbamat, N-Trichlormethylthiophthalimid und N-Trichlormethylthio-tetrahydrophthalimid als Fungizide in der Landwirtschaft und im Garten- und Weinbau verwendet werden können. Die genannten Verbindungen sind gute Mittel zur Bekämpfung von durch Fungi-Befall verursachten Krankheiten (R. Wegler, Chemie der Pflanzenschutz- und Schädlingsbekämpfungsmittel, Band 2, Seite 65 bis 66 und 109 und Band 4, Seiten 139 und 191, Springer-Verlag, Berlin/Heidelberg/New York (1970 und 1977)).

Jedoch sind diese Fungizide nicht nach erfolgter Infektion verwendbar, und ihre Wirkung bei niedrigen Aufwandkonzentrationen genügt nicht den Anforderungen der Praxis.

Es wurde nun gefunden, daß N-Alkinyl-naphthylamide der Formel I

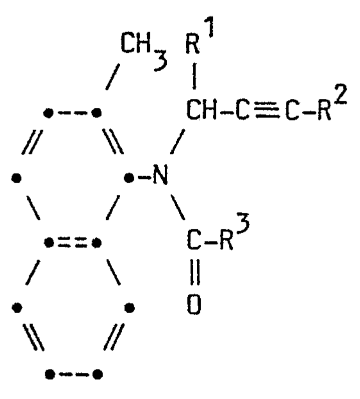

I,

Sws/P

worin
$R^1$ für Wasserstoff; $C_1$-$C_6$-Alkyl, $C_3$-$C_6$-Cycloalkyl und $C_2$-$C_4$--Alkenyl steht,
$R^2$ Wasserstoff oder Halogen bedeutet und
$R^3$ einen gegebenenfalls durch Methyl substituierten 2-Furyl-, 3-, 4- oder 5-Isoxazolyl-, 2-, 4- oder 5-Oxazolylrest oder einen gegebenenfalls durch Halogen, Nitro oder Methyl substituierten 3-, 4- oder 5-Isothiazolyl-, 2-, 4- oder 5-Thiazolyl- oder 3-(1,2,4-Oxadiazolyl)-, 3- oder 5-(1,2,4-Thiadiazolyl)-, 3-(1,2,5-Thiadiazolyl)- oder 2-(1,3,4-Thiadiazolyl)-Rest oder einen gegebenenfalls durch Halogen, $C_1$-$C_4$-Alkoxy-, $C_1$-$C_4$-Alkylthio-, 1-Imidazolyl-, 1-Pyrazolyl- oder 1,2,4-Triazolyl-(1)-substituierten $C_1$-$C_5$-Alkylrest oder die Gruppe $-CH_2-Y-R^4$ bedeutet, wobei Y Sauerstoff oder Schwefel ist und $R^4$ einen $C_1$-$C_6$-Alkoxy-ethyl- oder einen $C_1$-$C_6$-Alkoxy-ethoxy-ethylrest bedeutet oder ferner $R^3$ einen $C_2$-$C_5$-Alkenyl-, $C_2$-$C_5$-Alkinyl-, $C_1$-$C_5$-Alkoxy-, $C_1$-$C_5$-Alkoxycarbonyl- oder einen $C_3$-$C_7$--Cycloalkylrest bedeutet, starke fungizide Eigenschaften aufweisen.

In der Formel I steht $R^1$ bevorzugt für Wasserstoff, Methyl, Ethyl, n-Propyl, i-Propyl, Cyclopropyl, Vinyl und Alkyl, $R^2$ für Wasserstoff, Brom und Iod und $R^3$ bevorzugt für 2-Furyl, 3- oder 5-Isothiazolyl, 2-, 4- oder 5-Thiazolyl, 3- oder 5-Isoxazolyl, 2-, 4- oder 5-Oxazolyl, 3-(1,2,4-Oxadiazolyl), 4- oder 5-(1,2,3-Thiadiazolyl), 4-(1,2,5-Thiadiazolyl), Methoxymethyl, Ethoxymethyl, Methylthiomethyl, Ethylthiomethyl, Methoxyethyl, 1-Imidazolylmethyl, 1-Pyrazolylmethyl, 1-(1,2,4-Triazolyl)-methyl, Methyl, Ethyl, n-Propyl, Chlormethyl, Brommethyl, 1-Chlorethyl, 2-Chlorethyl, 1-Chlorpropyl, 4-Chlorbutyl, Vinyl, Propenyl, Ethinyl, Methoxy, Ethoxy, Propoxy, Methoxycarbonyl, Ethoxycarbonyl, Propoxycarbonyl, Cyclopropyl und Cyclohexyl,

wobei $R^4$ bevorzugt für Methoxyethoxy, Ethoxyethoxy, Methoxyethoxyethoxy und Butoxyethoxyethoxy steht.

Die neuen N-Alkyl-naphthylamide der Formel I besitzen ein chirales Kohlenstoffatom, wenn $R^1$ verschieden ist von Wasserstoff. Nach üblichen Methoden können die optisch reinen Enantiometeren erhalten werden. Als Fungizide sind sowohl die reinen Enantiomeren als auch die bei der Synthese üblicherweise anfallenden Racemate wirksam.

Weiterhin wurde gefunden, daß man die neuen Verbindungen der Formel I erhält, wenn man ein 2-Methylnaphthylamid der Formel II

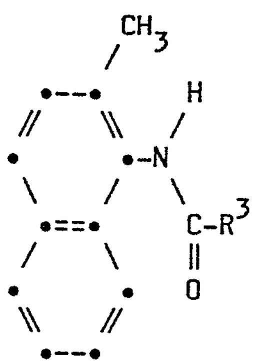

II,

worin
$R^3$ die oben angegebene Bedeutung hat, mit einem Alkin der Formel III

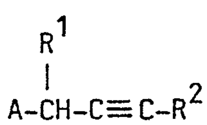

III,

worin
$R^1$ und $R^2$ die oben angegebenen Bedeutungen haben und A für eine nucleophil verdrängbare Abgangsgruppe steht, umsetzt

und die so erhaltenen N-Alkinyl-naphthylamide, falls $R^2$ ein Wasserstoffatom bedeutet, gegebenenfalls halogeniert.

In Formel III kommen als nucleofuge Abgangsgruppen für A beispielsweise Halogen - wie Chlor, Brom oder Iod -, Alkylsulfonyloxy- oder Arylsulfonyloxyreste - wie Methansulfonyloxy, Ethansulfonyloxy, Benzolsulfonyloxy- oder der p-Toluolsulfonyloxyrest in Betracht.

Die Umsetzung kann in Gegenwart eines Lösungs- oder Verdünnungsmittels, ein- oder zweiphasig, durchgeführt werden. Zu den bevorzugten Lösungs- bzw. Verdünnungsmitteln gehören Wasser, Halogenkohlenwasserstoffe - beispielsweise Methylenchlorid, Chloroform, 1,2-Dichlorethan, Chlorbenzol -, aliphatische oder aromatische Kohlenwasserstoffe - wie n-Pentan, Heptan, Petrolether, Cyclohexan, Benzol, Toluol oder Xylole -, Ester - wie Essigsäureethylester -, Nitrile - wie Acetonitril -, Sulfoxide - wie Dimethylsulfoxid -, Ketone - wie Aceton oder Methylethylketon -, Ether - wie Diethylether, Methyl-tert.-butylether, Tetrahydrofuran oder Dioxan - oder Gemische dieser Lösungsmittel. Zweckmäßig verwendet man das Lösungs- bzw. Verdünnungsmittel in einer Menge von 100 bis 2000 Gew.%, vorzugsweise 100 bis 1000 Gew.%, bezogen auf die Ausgangsstoffe der Formel II bzw. III.

Geeignete anorganische oder organische Basen, die gegebenenfalls als säurebindende Mittel dem Reaktionsgemisch zugesetzt werden können, sind beispielsweise Alkalihydroxide - wie Natriumhydroxid oder Kaliumhydroxid -, Alkalicarbonate - wie Natriumhydrogencarbonat, Kaliumhydrogencarbonat, Natriumcarbonat oder Kaliumcarbonat -, Alkalihydride - wie Natriumhydrid - oder tertiäre Amine - wie N,N-Diethylanilin, 1,4-Diazabicyclo[2.2.2]octan,

1,5-Diazabicyclo[4.3.0]non-5-en oder 1,5-Diazabicyclo-[5.4.0]-undec-7-en. Es können aber auch andere übliche Basen verwendet werden.

Mit Reaktionsbeschleunigern kann die Umsetzung stark beschleunigt werden. Als solche kommen vorzugsweise Metallhalogenide - wie Natriumbromid, Natriumiodid, Kaliumbromid oder Kaliumiodid - oder Kronenether - wie 12-Krone-4, 15-Krone-5, 18-Krone-6, Dibenzo-18-krone-6 oder Dicyclohexano-18-krone-6 - in Frage.

Führt man die Reaktion in Gegenwart von Phasentransfer-Katalysatoren, wie beispielsweise Benzyltriethylammoniumchlorid, Tetrabutylammonium-methosulfat oder Tetrapentylphosphoniumbromid durch, so kann man auch wäßrige Laugen - wie beispielsweise 50%ige Natronlauge - verwenden.

Diese Umsetzungen werden bei Temperaturen von etwa 0 bis 130°C, vorzugsweise +10 bis +50°C, durchgeführt.

Die Verbindungen der Formel III sind bekannt. Die Verbindungen der Formel II sind ebenfalls bekannt und lassen sich nach üblichen Verfahren herstellen.

Die folgenden Beispiele schildern die Herstellung der neuen Substanzen.

Beispiel 1

a) Herstellung des Ausgangsmaterials

Einer Lösung von 15,7 g (0,1 Mol) 2-Methyl-1-naphthylamin und 10,1 g (0,1 Mol) Triethylamin in 150 ml Toluol werden bei +5 bis o15°C 10,9 g (0,1 Mol) Methoxyacetylchlorid unter Rühren zugetropft. Das Ge-

misch wird bei 25°C weitere 4 h nachgerührt und mit 100 ml Eiswasser versetzt. Die organische Phase wird abgetrennt, zweimal mit je 50 ml Wasser gewaschen, über Natriumsulfat getrocknet und im Vakuum eingeengt. Der ölige Rückstand wird nach Zugabe von 100 ml Petrolether fest. Man erhält 18 g (78,6 %) N-Methoxyessigsäure-2-methyl-1-naphthylamid als farblose Kristalle, Fp. 68 bis 71°C.

b) Herstellung des Endproduktes

Einer Lösung von 17,9 g (0,078 Mol) N-Methoxyessigsäure-2-methyl-1-naphthylamid in 150 ml Toluol werden nacheinander 40 g 50%ige Natronlauge und 0,5 g Benzyltriethylammoniumchlorid zugegeben und anschließend unter heftigem Rühren 12,1 g (0,101 Mol) Propargylbromid zugetropft. Nach Abklingen der exothermen Reaktion (Temperaturanstieg von 20 auf 32°C) wird das Gemisch weitere 5 h nachgerührt, mit 50 ml Eiswasser verdünnt, die organische Phase abgetrennt, viermal mit je 50 ml Wasser gewaschen, über Natriumsulfat getrocknet und im Vakuum eingeengt. Der ölige Rückstand kristallisiert nach Zugabe von 70 ml Petrolether. Man erhält 18 g (86,4 %) N-Propargyl-N-methoxyessigsäure-2-methyl-1-naphthylamid als weiße Kristalle, Fp. 87 bis 89°C.

Beispiel 2

Einer Kaliumhypobromit-Lösung aus 28,1 g (0,5 Mol) Kaliumhydroxid und 16 g (0,1 Mol) Brom in 120 ml Wasser wird bei 0°C die Lösung von 13,4 g (0,05 Mol) N-Propargyl-N-methoxyessigsäure-2-methyl-1-naphthylamid in 350 ml Ether und 50 ml Tetrahydrofuran unter starkem Rühren zugetropft. Nach 16 h Rühren bei 25°C wird die organische Phase abgetrennt,

dreimal mit je 50 ml Wasser gewaschen, über Natriumsulfat getrocknet und im Vakuum eingeengt. Der Rückstand wird zusätzliche 3 h bei 50°C und 0,2 mbar getrocknet. Man erhält 15,5 (89,5 %) N-Brompropargyl-N-methoxyessigsäure-2-methyl-1-naphthylamin als hellbraunes Öl.

IR-Spektrum (KBr): 3045, 2920, 2212, 1720, 1417, 1396, 1255, 1195, 1130, 930, 818, 788 und 746 $cm^{-1}$.

In entsprechender Weise können folgende Verbindungen hergestellt werden.

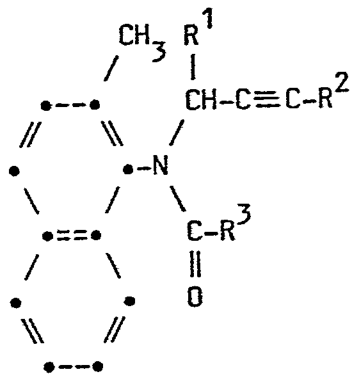

| Nr. | $R^1$ | $R^2$ | $R^3$ | Physikal. Konstante oder IR-Spektrum [$cm^1$] [KBr] |
|---|---|---|---|---|
| 3 | H | I | $-CH_2-O-CH_3$ | |
| 4 | $-CH_3$ | H | $-CH_2-O-CH_3$ | |
| 5 | $-CH_3$ | Br | $-CH_2-O-CH_3$ | öl |
| 6 | $-C_2H_5$ | H | $-CH_2-O-CH_3$ | öl |
| 7 | 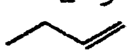 | H | $-CH_2-O-CH_3$ | öl |
| 8 | $-CH_3$ | H | $CH_3$<br>$-CH-O-CH_3$ | Harz 2983, 2928, 1669, 1395, 1380, 1266, 1212, 1124, 816, 790, 755. |
| 9 | H | H | $-CH_2-O-(CH_2CH_2O)_2CH_3$ | Harz 3040, 2915, 2870, 2110, 1742, 1396, 1255, 1115, 1020, 818, 788, 746. |
| 10 | $-CH_3$ | H | $-CH_2-O-(CH_2CH_2O)_2CH_3$ | Harz |
| 11 | $-CH_3$ | H | $-CH_2-O-CH_2-CH_2-O-C_2H_5$ | öl |
| 12 | H | H | $-CH_2-Cl$ | Fp. 84-86°C |
| 13 | $-CH_3$ | H | $-CH_2-Cl$ | Harz |
| 14 | $-CH_3$ | H | $-CH_2-Br$ | Harz |
| 15 | 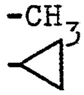 | H | $-CH_2-Br$ | öl |
| 16 | $-CH_3$ | H | $-C_2H_5$ | Harz 2983, 2938, 1663, 1461, 1377, 1265, 1135, 1030, 816, 786, 750. |

| Nr. | $R^1$ | $R^2$ | $R^3$ | Physikal. Konstante oder IR-Spektrum [cm$^{1}$] [KBr] |
|---|---|---|---|---|
| 17 | $-CH_3$ | H | $-CH(CH_3)_2$ | Harz 3042, 2970, 1700, 1385, 1367, 1265, 1235, 1090, 815, 785, 747. |
| 18 | $-CH_3$ | H | 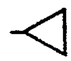 | |
| 19 | $-CH_3$ | H | $-CH=CH-CH_3$ | |
| 20 | $-CH_3$ | H | $-\overset{Cl}{C}H-CH_3$ | Öl |
| 21 | $-CH_3$ | H | $-CH_2-CH_2-Cl$ | |
| 22 | H | H | 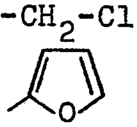 | Fp. 121-123°C |
| 23 | $-CH_3$ | H | " | Öl 3060, 2990, 2930, 2120, 1641, 1508, 1471, 1397, 1340, 1186, 820, 758. |
| 24 | H | H | 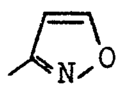 | Fp. 90-92°C |
| 25 | $-CH_3$ | H | " | Harz 3060, 2990, 1661, 1520, 1509, 1450, 1421, 1339, 1200, 1035, 815, 772. |
| 26 | $-CH_3$ | Br | " | Harz |
| 27 | H | H | 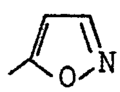 | Harz |
| 28 | $-CH_3$ | H | " | Harz |
| 29 | H | H | 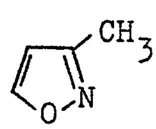 | Fp. 109-110°C |
| 30 | H | H | 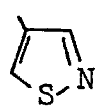 | Öl |
| 31 | $-CH_3$ | H | 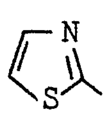 | |

| Nr. | $R^1$ | $R^2$ | $R^3$ | Physikal. Konstante oder IR-Spektrum [$cm^1$] [KBr] |
|---|---|---|---|---|
| 32 | $-CH_3$ | H | 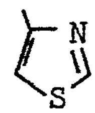 | |
| 33 | H | H | 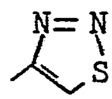 | Fp. 132-135°C |
| 34 | $-CH_3$ | H | " | Harz |
| 35 | $-CH_3$ | H | 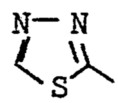 | Harz |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podopshaera leucotricha an Äpfeln, Sphaerotheca fuliginea an Rosen, Erysiphe cichoriacearum an Gurken.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes

zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Darüber hinaus sind viele der neuen Verbindungn systemisch wirksam, so daß über die Wurzelbehandlung auch ein Schutz oberirdischer Pflanzenteile möglich ist.

Die neuen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethylthio-phthalimid
5-Ethoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid

2,5-Dimethyl-furan-3-carbonsäurecyclohexylamid
N-Cyclohexyl-N-methoxy-2,5-dimethyl-furan-3-carbonsäure-amid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Wirkstoffen kombiniert werden können sind beispielsweise:

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat) und
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat

heterocyclische Strukturen, wie
2-Heptadecyl-2-imidazolin-acetat
2,4-Dichlor-6-(o-chloranilino)-s-triazin
O,O-Diethyl-phthalimidophosphonothioat

5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-di-yl-bis-(1-(2,2,2-trichlor-ethyl)-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Iod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)-2-butanon

1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet. Die Aufwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder

Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol, Alkylphenolpolyglykolether, Tributylphenylpolyglykolether, Alkalarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind

z.B. Mineralerden wie Silicagel, Kieselsäuren, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Beispiele für Zubereitungen sind:

I. Man vermischt 90 Gew.-Teile der Verbindung 1 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-mono-ethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung 8 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung 8 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung 11 werden mit 3 Gew.--Teilen des Natriumsalzes der Diisobutylnaphthalin--alpha-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung 18 werden mit 97 Gew.--Teilen feinteiligem Kaolin innig vermischt. Man erhält auf diese Weise ein Stäubemittel, das 3 Gew.-% des Wirkstoffs enthält.

VII. 30 Gew.-Teile der Verbindung 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 25 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Als bekannte Vergleichswirkstoffe wurden die folgenden Verbindungen verwendet:
N-Trichlormethylthiophthalimid, N-Trichlormethylthiotetrahydrophthalimid, Zink-ethylen-1,2-bis-dithiocarbamat.

Beispiel A

Wirksamkeit gegen Plasmopara viticola

Blätter von den Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporenangienträgerausbruches abermals für 16 Stunden in der feuchten Kammer aufgestellt. Dann erfolgt die Beurteilung des Ausmaßes des Pilzausbruches auf den Blattunterseiten.

Beispiel B

Wirksamkeit gegen Phytophthora infestans an Tomaten

Blätter von Topfpflanzen der Sorte "Große Fleichtomate" werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

In den Beispielen A und B zeigten beispielsweise die Substanzen der Beispiele 1, 4, 24 und 29 bei der Anwendung als 0,025%ige Spritzbrühen eine bessere Wirkung (beispielsweise 97 %) als die Vergleichssubstanzen (beispielsweise 80 %).

## Patentansprüche

1. N-Alkinyl-naphthylamide der allgemeinen Formel I

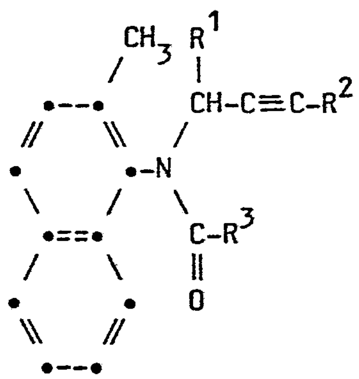

I,

worin
$R^1$ für Wasserstoff; $C_1$-$C_6$-Alkyl, $C_3$-$C_6$-Cycloalkyl und $C_2$-$C_4$-Alkenyl steht,
$R^2$ Wasserstoff oder Halogen bedeutet und
$R^3$ einen gegebenenfalls durch Methyl substituierten 2-Furyl-, 3-, 4- oder 5-Isoxazolyl-, 2-, 4- oder 5-Oxazolylrest oder einen gegebenenfalls durch Halogen, Nitro oder Methyl substituierten 3-, 4- oder 5-Isothiazolyl-, 2-, 4- oder 5-Thiazolyl- oder 3-(1,2,4-Oxadiazolyl)-, 3- oder 5-(1,2,4-Thiadiazolyl)-, 3-(1,2,5-Thiadiazolyl)- oder 2-(1,3,4-Thiadiazolyl)--Rest oder einen gegebenenfalls durch Halogen, $C_1$-$C_4$-Alkoxy-, $C_1$-$C_4$-Alkylthio-, 1-Imidazolyl-, 1-Pyrazolyl- oder 1,2,4-Triazolyl-(1)-substituierten $C_1$-$C_5$-Alkylrest oder die Gruppe $-CH_2-Y-R^4$ bedeutet, wobei Y Sauerstoff oder Schwefel ist und $R^4$ einen $C_1$-$C_6$-Alkoxyethyl- oder einen $C_1$-$C_6$-Alkoxy-ethoxy--ethylrest bedeutet oder ferner $R^3$ einen $C_2$-$C_5$--Alkenyl-, $C_2$-$C_5$-Alkinyl-, $C_1$-$C_5$-Alkoxy-, $C_1$-$C_5$--Alkoxycarbonyl- oder einen $C_3$-$C_7$-Cycloalkylrest bedeutet.

2. Alkinyl-naphthylamid gemäß Anspruch 1, dadurch gekennzeichnet, daß $R^1$ Wasserstoff oder Methyl, daß $R^2$ Wasserstoff, Brom oder Iod und daß $R^3$ 2-Furyl, 3- oder 5-Isothiazolyl, 2-, 4- oder 5-Thiazolyl, 3- oder 5-Isoxazolyl, 2-, 4- oder 5-Oxazolyl, 3-(1,2,4-Oxadiazolyl), 4- oder 5-(1,2,3-Thiadiazolyl), 4-(1,2,5-Thiadiazolyl), Methoxy- oder Ethoxymethyl, Methylthio- oder Ethylthiomethyl, Methoxyethyl, 1-Imidazolylmethyl, 1-Pyrazolylmethyl, 1-(1,2,4-Triazolyl)-methyl, Methyl, Ethyl, n-Propyl, Chlormethyl, Brommethyl, 1-Chlorethyl, 2-Chlorethyl, 1-Chlorpropyl, 4-Chlorbutyl, Vinyl, Propenyl, Ethinyl, Methoxy, Ethoxy, Propoxy, Methoxycarbonyl, Ethoxycarbonyl, Propoxycarbonyl, Cyclopropyl und Cyclohexyl und $R^4$ Methoxyethoxy, Ethoxyethoxy, Methoxyethoxyethoxy und Butoxyethoxyethoxy bedeutet.

3. Fungizid, enthaltend ein N-Alkinyl-naphthylamid der Formel I gemäß Anspruch 1.

4. Fungizid, enthaltend eine Verbindung gemäß Anspruch 2.

5. Fungizid, enthaltend ein N-Alkinyl-naphthylamid der Formel I gemäß Anspruch 1 und einen inerten Trägerstoff.

6. Verfahren zur Herstellung von Verbindungen gemäß Anspruch 1, dadurch gekennzeichnet, daß man ein 2-Methylnaphthylamid der Formel II

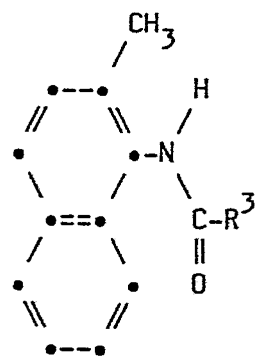

II,

worin
$R^3$ die in Anspruch 1 angegebene Bedeutung hat, mit einem Alkin der Formel III

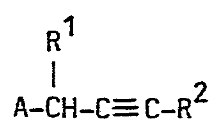

III,

worin
$R^1$ und $R^2$ die in Anspruch 1 angegebenen Bedeutungen haben und A für eine nucleophil verdrängbare Abgangsgruppe steht, umsetzt und die so erhaltenen N-Alkinyl-naphthylamide, falls $R^2$ ein Wasserstoffatom bedeutet, gegebenenfalls anschließend halogeniert.

7. Verfahren zur Bekämpfung von Pilzen, dadurch gekennzeichnet, daß man eine fungizid wirksame Menge eines N-Alkinyl-naphthylamids der Formel I gemäß Anspruch 1 auf die Pilze oder durch Pilzbefall bedrohte Flächen, Pflanzen und Saatgüter einwirken läßt.

Patentansprüche (für Österreich)

1. Fungizid, enthaltend ein N-Alkinyl-naphthylamid der allgemeinen Formel I

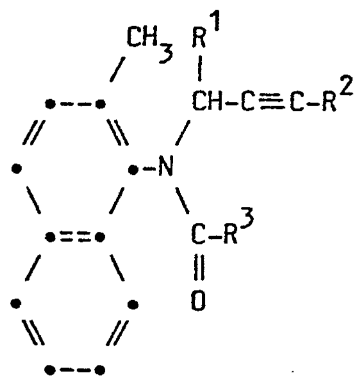

I,

worin
$R^1$ für Wasserstoff; $C_1$-$C_6$-Alkyl, $C_3$-$C_6$-Cycloalkyl und $C_2$-$C_4$-Alkenyl steht,
$R^2$ Wasserstoff oder Halogen bedeutet und
$R^3$ einen gegebenenfalls durch Methyl substituierten 2-Furyl-, 3-, 4- oder 5-Isoxazolyl-, 2-, 4- oder 5-Oxazolylrest oder einen gegebenenfalls durch Halogen, Nitro oder Methyl substituierten 3-, 4- oder 5-Isothiazolyl-, 2-, 4- oder 5-Thiazolyl- oder 3-(1,2,4-Oxadiazolyl)-, 3- oder 5-(1,2,4-Thiadiazolyl)-, 3-(1,2,5-Thiadiazolyl)- oder 2-(1,3,4-Thiadiazolyl)--Rest oder einen gegebenenfalls durch Halogen, $C_1$-$C_4$-Alkoxy-, $C_1$-$C_4$-Alkylthio-, 1-Imidazolyl-, 1-Pyrazolyl- oder 1,2,4-Triazolyl-(1)-substituierten $C_1$-$C_5$-Alkylrest oder die Gruppe $-CH_2-Y-R^4$ bedeutet, wobei Y Sauerstoff oder Schwefel ist und $R^4$ einen $C_1$-$C_6$-Alkoxyethyl- oder einen $C_1$-$C_6$-Alkoxy-ethoxy--ethylrest bedeutet oder ferner $R^3$ einen $C_2$-$C_5$--Alkenyl-, $C_2$-$C_5$-Alkinyl-, $C_1$-$C_5$-Alkoxy-, $C_1$-$C_5$--Alkoxycarbonyl- oder einen $C_3$-$C_7$-Cycloalkylrest bedeutet.

2. Fungizid gemäß Anspruch 1, <u>dadurch gekennzeichnet</u>, daß $R^1$ Wasserstoff oder Methyl, daß $R^2$ Wasserstoff, Brom oder Jod und daß $R^3$ 2-Furyl, 3- oder 5-Isothiazolyl, 2-, 4- oder 5-Thiazolyl, 3- oder 5-Isoxazolyl, 2-, 4- oder 5-Oxazolyl, 3-(1,2,4-Oxadiazolyl), 4- oder 5-(1,2,3-Thiadiazolyl), 4-(1,2,5-Thiadiazolyl), Methoxy- oder Ethoxymethyl, Methylthio- oder Ethylthiomethyl, Methoxyethyl, 1-Imidazolylmethyl, 1-Pyrazolylmethyl, 1-(1,2,4-Triazolyl)-methyl, Methyl, Ethyl, n-Propyl, Chlormethyl, Brommethyl, 1-Chlorethyl, 2-Chlorethyl, 1-Chlorpropyl, 4-Chlorbutyl, Vinyl, Propenyl, Ethinyl, Methoxy, Ethoxy, Propoxy, Methoxycarbonyl, Ethoxycarbonyl, Propoxycarbonyl, Cyclopropyl und Cyclohexyl und $R^4$ Methoxyethoxy, Ethoxyethoxy, Methoxyethoxyethoxy und Butoxyethoxyethoxy bedeutet.

3. Fungizid, enthaltend ein N-Alkinyl-naphthylamid der Formel I gemäß Anspruch 1 und einen inerten Trägerstoff.

4. Verfahren zur Herstellung von Verbindungen gemäß Anspruch 1, <u>dadurch gekennzeichnet</u>, daß man ein 2-Methylnaphthylamid der Formel II

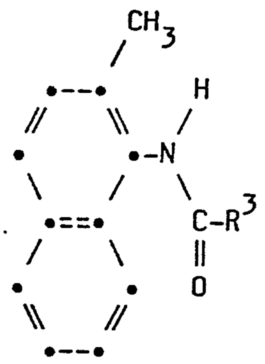

II,

worin
$R^3$ die in Anspruch 1 angegebene Bedeutung hat, mit einem Alkin der Formel III

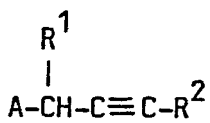

III,

worin
$R^1$ und $R^2$ die in Anspruch 1 angegebenen Bedeutungen haben und A für eine nucleophil verdrängbare Abgangsgruppe steht, umsetzt und die so erhaltenen N-Alkinyl-naphthylamide, falls $R^2$ ein Wasserstoffatom bedeutet, gegebenenfalls anschließend halogeniert.

5. Verfahren zur Bekämpfung von Pilzen, dadurch gekennzeichnet, daß man eine fungizid wirksame Menge eines N-Alkinyl-naphthylamids der Formel I gemäß Anspruch 1 auf die Pilze oder durch Pilzbefall bedrohte Flächen, Pflanzen und Saatgüter einwirken läßt.